# EUROPEAN PATENT APPLICATION

(11) **EP 4 039 773 A1**
(43) Date of publication of application: **10.08.2022**
(21) Application number: 20872841.0
(22) Date of filing: 16.09.2020
(51) Int. Cl.: C09K 11/06, H01L 51/50

(54) **ORGANIC ELECTROLUMINESCENT ELEMENT**

(30) Priority: 30.09.2019 JP 2019180020
(71) Applicant: NIPPON STEEL Chemical & Material Co., Ltd., Tokyo 103-0027 (JP)
(72) Inventor: OGAWA Junya, Tokyo 103-0027 (JP); KITAHARA Ikumi, Tokyo 103-0027 (JP)
(74) Representative: Vossius & Partner Patentanwälte Rechtsanwälte mbB
(86) International application number: PCT/JP2020/034992
(87) International publication number: WO 2021/065491

(57) **Abstract**

An organic electroluminescent device having high efficiency and high driving stability, and a material suitable for the organic electroluminescent device are provided. The material for organic electroluminescent devices of the present invention is an oligopyridine compound represented by the following general formula (1), and the organic electroluminescent device of the present invention includes the oligopyridine compound advantageously in a light emitting layer or a hole blocking layer. In the formula, R and R' each are hydrogen, an aliphatic hydrocarbon group, an aromatic hydrocarbon group, or an aromatic heterocyclic group; at least one of X is N; and a+b+c≥3.

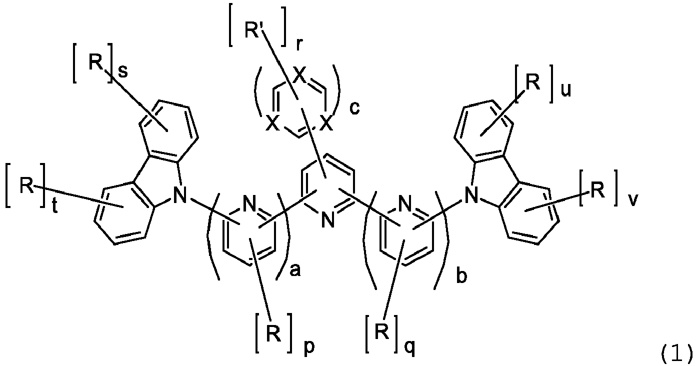

## Description

### Technical Field

The present invention relates to an organic electroluminescent device (referred to as an organic EL device). More specifically, the present invention relates to a material for organic electroluminescent devices made of an oligopyridine compound, and an organic electroluminescent device made therefrom.

### Background Art

By applying a voltage to an organic electroluminescent device, holes are injected from an anode and electrons are injected from a cathode into a light emitting layer. Then, in the light emitting layer, the injected holes and electrons are recombined to generate excitons. On this occasion, singlet excitons and triplet excitons are generated at a ratio of 1:3 according to the statistical law of electron spin. It is said that the internal quantum efficiency of a fluorescence emission type organic electroluminescent device that uses light emission by singlet excitons is limited to 25%. On the other hand, it is known that the phosphorescence emission type organic electroluminescent device that uses light emission by triplet excitons can increase the internal quantum efficiency to 100% when intersystem crossing is efficiently performed from the singlet excitons.

However, extending the life of a phosphorescence emission type organic electroluminescent device has been a technical challenge.

Recently, high-efficiency organic electroluminescent devices using delayed fluorescence have been developed. For example, Patent Literature 1 discloses an organic electroluminescent device using a TTF (Triplet-Triplet Fusion) mechanism, which is one of the delayed fluorescence mechanisms. The TTF mechanism utilizes a phenomenon that singlet excitons are generated by the collision of two triplet excitons, and it is theoretically conceivable that the internal quantum efficiency can be increased to 40%. However, since the efficiency is lower than that of a phosphorescence emission type organic electroluminescent device, further improvement in efficiency is required.

Patent Literature 2 discloses an organic electroluminescent device using a TADF (Thermally Activated Delayed Fluorescence) mechanism. The TADF mechanism utilizes a phenomenon of inverse intersystem crossing from triplet excitons to singlet excitons that occurs in materials with a small energy difference between singlet and triplet levels, and it is theoretically conceivable that the internal quantum efficiency can be increased to 100%. However, as with the phosphorescence emission type device, further improvement in life characteristics is required.

### Citation List

### Patent Literature

Patent Literature 1: WO2010/134350A
Patent Literature 2: WO2011/070963A
Patent Literature 3: JP2006-232813A
Patent Literature 4: KR2017-0114445A
Patent Literature 5: WO2012/115219A
Patent Literature 6: KR2014-0028640A
Patent Literature 7: CN102503937A

Patent Literature 3, 4, and 5 disclose the use of a bipyridine compound as host material.

Patent Literature 6 discloses the use of a terpyridine compound as a host material. Patent Literature 7 discloses the use of a quaterpyridine compound as a host material. However, none of them can be said to be sufficient, and further improvement is desired.

### Summary of Invention

In order to apply an organic electroluminescent device to a display device such as a flat panel display, it is necessary to improve the luminous efficiency of the device and at the same time to sufficiently ensure the stability during driving. In view of the above situation, it is an object of the present invention to provide a practically useful organic electroluminescent device having high efficiency and high driving stability and a compound suitable for the organic electroluminescent device.

As a result of diligent studies, the present inventors have found that an oligopyridine compound having at least four or more pyridine rings linked can be used for an organic electroluminescent device to exhibit excellent properties, and have completed the present invention.

The present invention is a material for organic electroluminescent devices, comprising an oligopyridine compound represented by the following general formula (1).

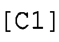

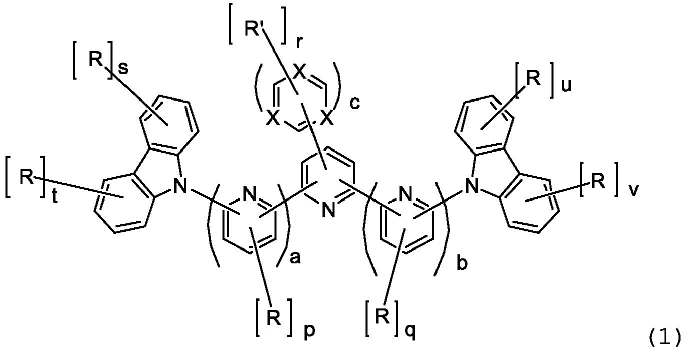

In the formula, R and R' each are independently hydrogen, deuterium, an aliphatic hydrocarbon group having 1 to 10 carbon atoms, a substituted or unsubstituted aromatic hydrocarbon group having 6 to 10 carbon atoms, or a substituted or unsubstituted aromatic heterocyclic group having 3 to 12 carbon atoms, and when R and R' each are an aromatic hydrocarbon group or an aromatic heterocyclic group, it may form a condensed ring with an adjacent ring. However, R' contains no 9-carbazole ring group. X independently represents N, C-R' or C-, and at least one is N. a, b and c each represent the number of repetitions and each independently represent an integer of 0 to 3, and a+b+c≤3. p, q, r, s, t, u and v each represent the number of substitutions and each independently represent an integer of 1 to 3.

The compound for organic electroluminescent devices represented by the general formula (1) includes compounds represented by formulas (2) to (4). 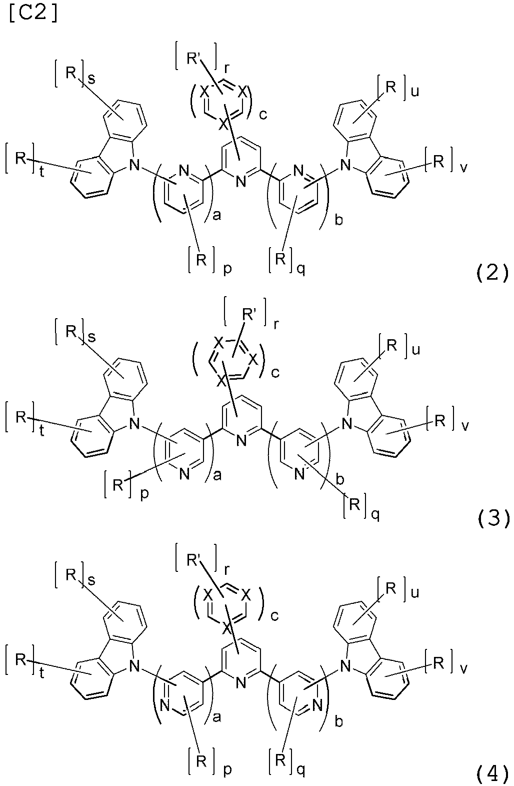 wherein X, R, R', a to c, and p to v are the same as in the general formula (1).

In the general formula (1) and formulas (2) to (4), it is desirable that a+b=3.

Further, the present invention relates to an organic electroluminescent device comprising an anode, organic layers and a cathode laminated on a substrate, wherein at least one layer of the organic layers is an organic layer containing the material for organic electroluminescent devices.

The organic layer containing the material for organic electroluminescent devices may be at least one layer selected from the group consisting of a light emitting layer, an electron transporting layer, and a hole blocking layer. Preferably, the organic layer is a light emitting layer containing a host material and a light emitting dopant material.

A preferred aspect of the organic electroluminescent device is shown below.

The ratio of a first host to the total of the first host and a second host is more than 20 wt% and less than 55wt%.

The light-emitting dopant material is an organic metal complex containing at least one metal selected from the group consisting of ruthenium, rhodium, palladium, silver, rhenium, osmium, iridium, platinum and gold, or a thermally activated delayed fluorescence emitting dopant.

A hole blocking layer is provided adjacent to the light emitting layer, and the hole blocking layer contains a compound represented by the general formula (1).

The material for organic electroluminescent devices of the present invention has a structure having four or more nitrogen-containing 6-membered rings including at least three or more pyridine rings, bonding to two or more carbazole rings. An oligopyridine compound having such structural characteristics has a lowest unoccupied molecular orbital (LUMO) that affects electron injection and transportation, widely distributed throughout the pyridine ring, so that the electron injection and transportation of the device can be controlled at a high level by changing the number and connection mode of the pyridine rings, as well as the type and number of substituents bonding to the pyridine rings. On the other hand, the lowest occupied molecular orbital (HOMO) that affects the hole injection and transportation is widely distributed on the carbazole ring, so that hole injection and transportation of the device can be controlled at a high level by changing the type and number of substituents on the carbazole group. Having such characteristics, the material of the present invention is excellent as a host material having a good balance in both charges (hole/electron) injection and transport characteristics. By using the material for organic electroluminescent devices, reduction in the drive voltage and high luminous efficiency of the device can be achieved.

### Brief Description of Drawing

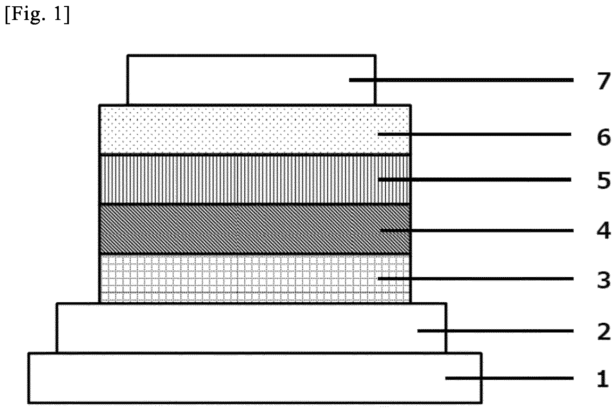

[Figure 1] Figure 1 is a cross-sectional structure view of an organic electroluminescent device.

### Description of Embodiment

The material for organic electroluminescent devices of the present invention is an oligopyridine compound represented by the general formula (1). Preferably, the material is a compound represented by any of the formulas (2) to (4). The oligopyridine compound has a structure having four or more nitrogen-containing 6-membered rings including at least three or more pyridine rings, bonding to two or more carbazole rings.

In the general formula (1) and the formulas (2) to (4), the common symbols have the same meaning.

R and R' each independently represent hydrogen, deuterium, an aliphatic hydrocarbon group having 1 to 10 carbon atoms, an aromatic hydrocarbon group having 6 to 10 carbon atoms, or aromatic heterocyclic group having 3 to 12 carbon atoms. An aliphatic hydrocarbon group having 1 to 8 carbon atoms, a phenyl group, or an aromatic heterocyclic group having 3 to 12 carbon atoms is preferred. However, R' contains no 9-carbazole ring group, and advantageously contains no group having a carbazole ring structure.

When R and R' are aromatic hydrocarbon groups or aromatic heterocyclic groups, a condensed ring may be formed with a nitrogen-containing 6-membered ring or a benzene ring to be substituted by R and R'.

More preferably, R is hydrogen, an aliphatic hydrocarbon group having 1 to 6 carbon atoms, a phenyl group, or a carbazole ring group, and R' is hydrogen, an aliphatic hydrocarbon group having 1 to 6 carbon atoms, or a phenyl group.

In the present specification, it is understood that an aromatic hydrocarbon group, an aromatic heterocyclic group or an aliphatic hydrocarbon group may have a substituent unless otherwise specified.

Specific examples of the aliphatic hydrocarbon group include methyl, ethyl, propyl, butyl, pentyl, hexyl, heptyl, octyl, nonyl, and decyl. An alkyl group having 1 to 4 carbon atoms is preferred.

Specific examples of the aromatic hydrocarbon group or aromatic heterocyclic group include an aromatic group formed by taking one H from benzene, naphthalene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isothiazole, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzoisothiazole, benzothiadiazole, dibenzofuran, dibenzothiophene, dibenzoselenophene, or carbazole. Preferred examples include an aromatic group formed from benzene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzoisothiazole, or benzothiazole. More preferred examples include an aromatic group formed from benzene, pyridine, pyrimidine, triazine, thiophene, isothiazole, thiazole, pyridazine, pyrrole, pyrazole, imidazole, triazole, thiadiazole, pyrazine, furan, isoxazole, oxazole, or oxadiazole.

In the formula, a, b and c each represent the number of repetitions and are integers of 0 to 3. Here, a+b+c is 3 or more, preferably 2. Further, preferably, a+b is 2.

X independently represents N, C-R' or C-, and at least one X is N. And it is preferable that only one X be N.

Further, when only one X is N, the compound represented by the general formula (1) has four or more pyridine rings.

Examples of preferred aspect of the compound represented by the general formula (1) include a compound represented by any of the formulas (2) to (4).

Although specific examples of the compound represented by the general formula (1) are shown below, the compound is not limited to these exemplified compounds.

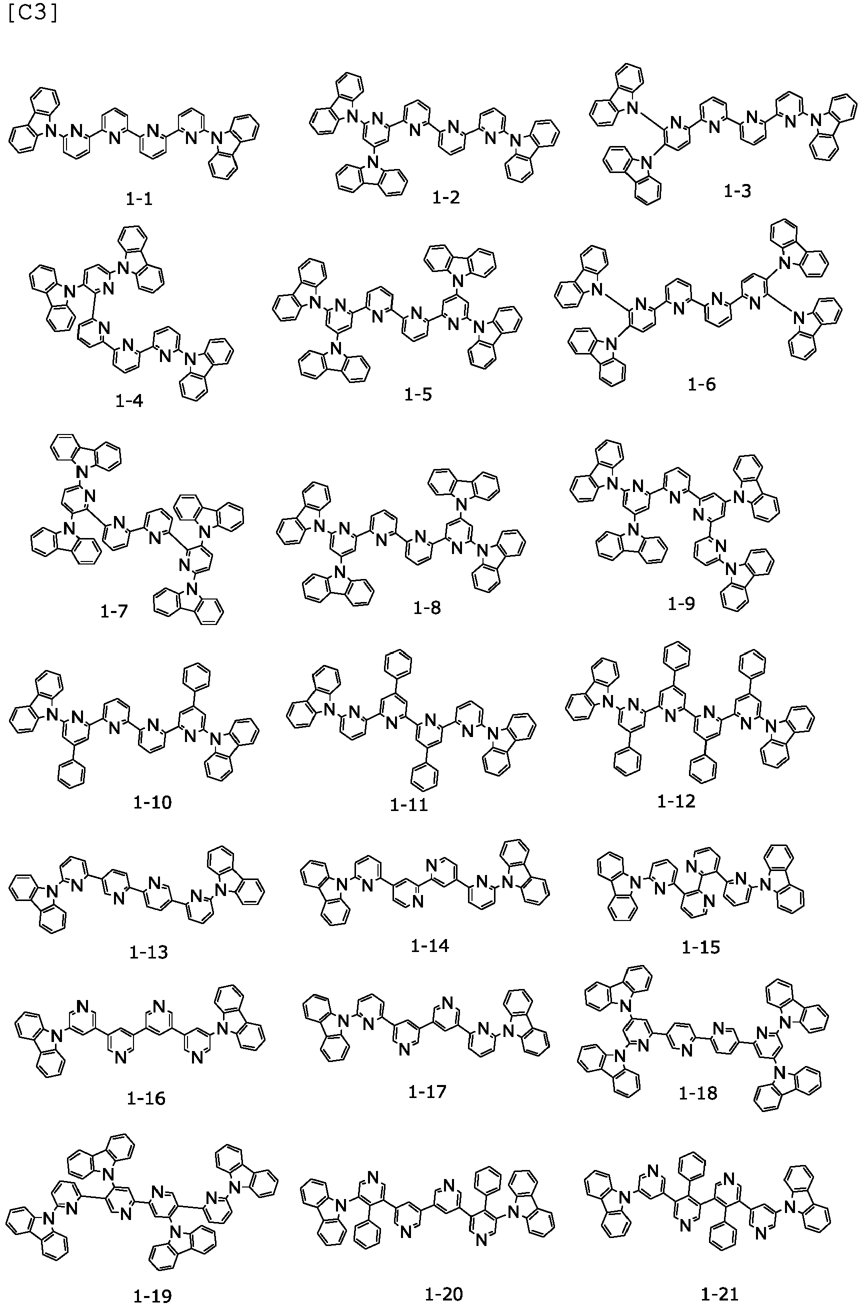

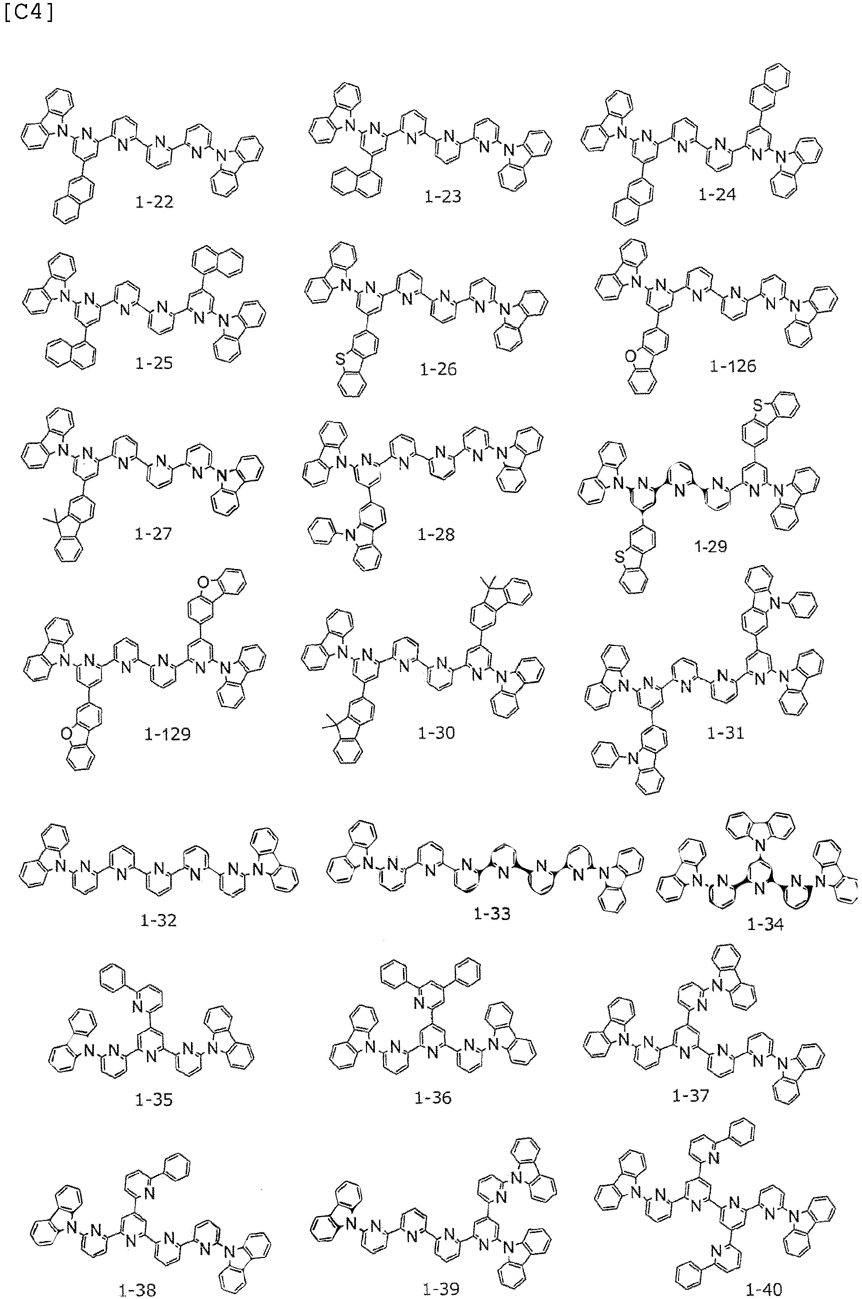

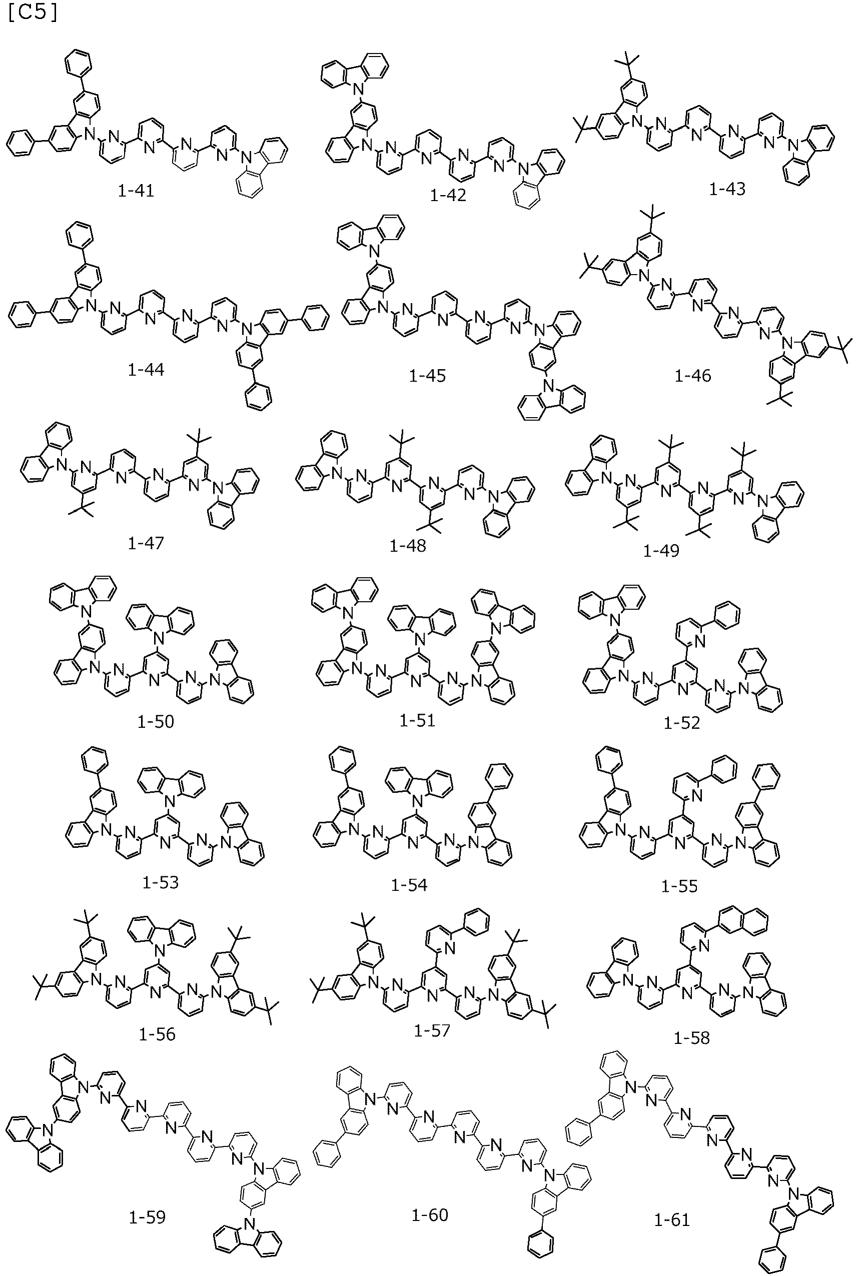

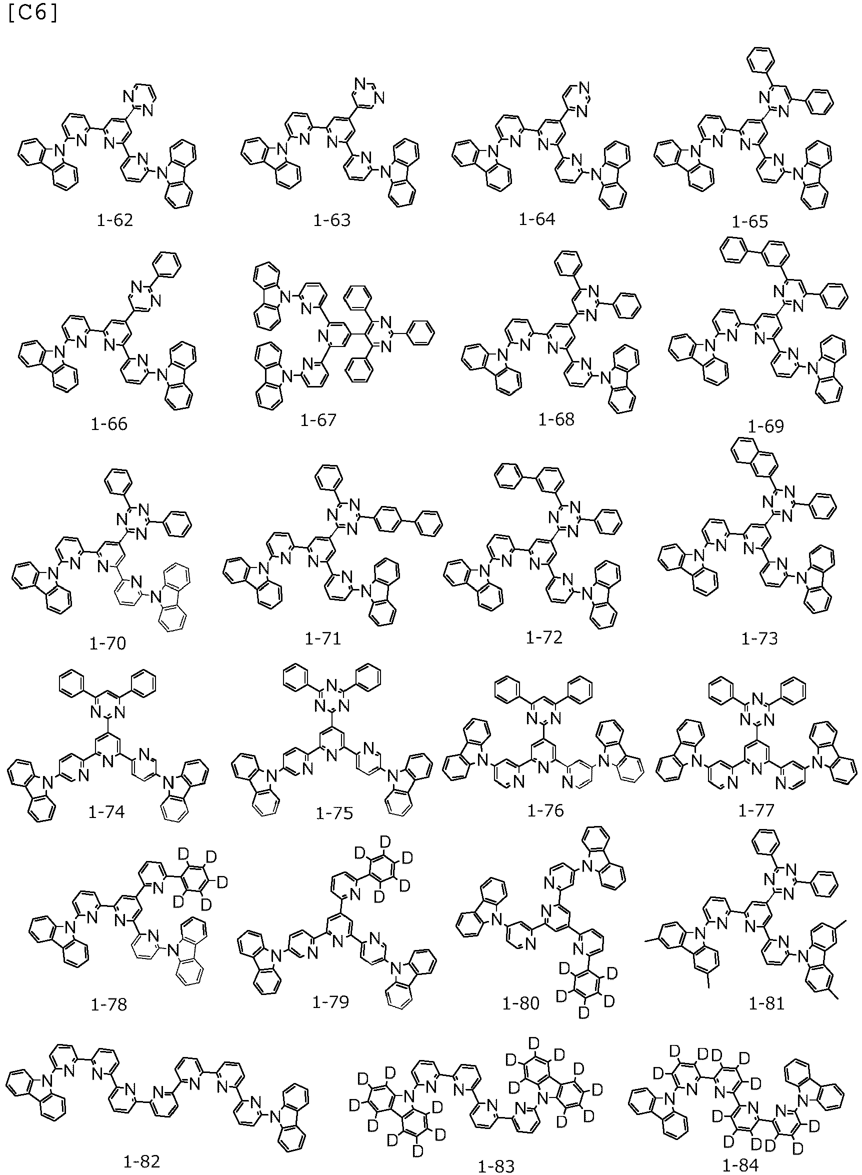

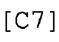

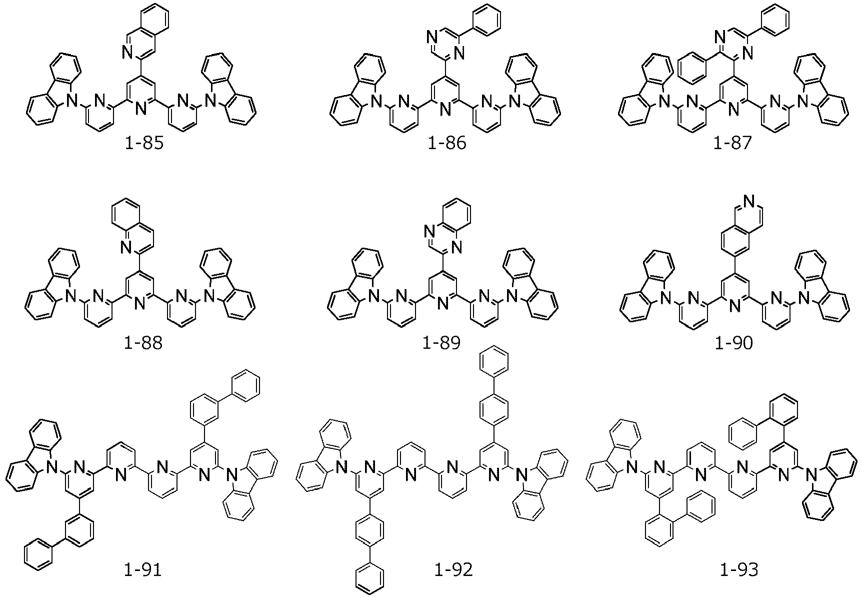

The material for organic electroluminescent devices of the present invention (also referred to as a compound of the present invention, a compound represented by the general formula (1) or an oligopyridine compound), is contained in at least one organic layer of an organic electroluminescent device including an anode, a plurality of organic layers and a cathode laminated on a substrate, so that an excellent organic electroluminescent device is provided. As the organic layer in which the material is contained, a light emitting layer, an electron transporting layer or a hole blocking layer is suitable. Here, when used for a light emitting layer, the compound of the present invention can be used as a host material of a light emitting layer containing a fluorescent, delayed fluorescence or phosphorescence emitting dopant, and also as an organic light emitting material that emits fluorescence and delayed fluorescence. It is particularly preferable that the compound of the present invention be contained as a host material of a light emitting layer containing a phosphorescence emitting dopant.

When the compound of the present invention is used as an organic light emitting material that emits fluorescence and delayed fluorescence (also referred to as a thermally activated delayed fluorescence emitting dopant material), it is preferable that another organic compound of which at least one of the excited singlet energy and the excited triple energy is higher than that of the compound of the present invention be used as the host material.

An organic electroluminescent device made from the material for organic electroluminescent devices of the present invention is described as follows.

The organic electroluminescence device of the present invention has an organic layer having at least one light emitting layer between an anode and a cathode laminated on a substrate, and at least the one organic layer contains the material for organic electroluminescent devices of the present invention. Advantageously, the material for organic electroluminescent devices of the present invention is contained together with a phosphorescence emitting dopant in the light emitting layer.

The structure of the organic electroluminescent device of the present invention is described with reference to drawing, though the structure of the organic electroluminescent device of the present invention is not limited to the one shown in the drawing.

Figure 1 is a cross-sectional structure view of a general organic electroluminescent device used in the present invention, wherein "1" represents a substrate, "2" represents an anode, "3" represents a hole injecting layer, "4" represents a hole transporting layer, "5" represents a light emitting layer, "6" represents an electron transporting layer, and "7" represents a cathode, respectively. The organic electroluminescent device of the present invention may have an exciton blocking layer adjacent to the light emitting layer, or may have an electron blocking layer between the light emitting layer and the hole injecting layer. The exciton blocking layer can be inserted into any of the anode side or the cathode side of the light emitting layer, or can be inserted into both at the same time. The organic electroluminescent device of the present invention has a substrate, an anode, a light emitting layer, and a cathode as essential layers, and preferably has a hole injecting and transporting layer and an electron injecting and transporting layer as layers other than the essential layers, further having a hole blocking layer between the light emitting layer and the electron injecting and transporting layer. The hole injecting and transporting layer means any one or both of a hole injecting layer and a hole transporting layer, and the electron injecting and transporting layer means any one or both of an electron injecting layer and an electron transporting layer.

An inverted structure of the structure shown in Figure 1 is possible, in which a cathode 7, an electron transporting layer 6, a light emitting layer 5, a hole transporting layer 4, and an anode 2 are laminated in this order on the substrate 1. In this case also, layers may be added or omitted on an as needed basis.

### -Substrate-

The organic electroluminescent device of the present invention is preferably supported by a substrate. The substrate is not particularly limited as long as it is conventionally used for organic electroluminescent devices, and for example, a substrate made of glass, transparent plastic, quartz, or the like may be used.

### -Anode-

As the anode in an organic electroluminescent device, a metal, an alloy, an electrically conductive compound or a mixture thereof, which are having a large work function (4 eV or more) is preferably used for an electrode material. Specific examples of the electrode material include metals such as Au, conductive transparent materials such as CuI, indium tin oxide (ITO), SnO₂, and ZnO. Alternatively, a material such as IDIXO(In₂O₃-ZnO), which is amorphous and from which a transparent conductive film can be made, may be used. An anode may be produced by forming a thin film through vapor deposition or sputtering of these electrode materials, and then forming a pattern having a desired shape through photolithography. Alternatively, when a precise pattern accuracy is not required (about 100 µm or more), the pattern may be formed through a mask having a desired shape during vapor deposition or sputtering of the electrode material. Alternatively, when an applicable substance such as organic conductive compound is used, a wet film forming method such as printing and coating may also be used. When light emission is taken out from the anode, it is desirable to increase the transmittance to more than 10%, and the sheet resistance as anode is preferably several hundreds **Ω**/sq or less. Further, the film thickness depends on the material, and is usually selected in the range of 10 to 1000 nm, preferably 10 to 200 nm.

### -Cathode-

On the other hand, as the cathode, an electrode material made of metal (referred to as an electron injectable metal), an alloy, an electrically conductive compound or a mixture thereof, which are having a small work function (4 eV or less) is used.

Specific examples of the electrode material include sodium, sodium-potassium alloy, magnesium, lithium, magnesium/copper mixture, magnesium/silver mixture, magnesium/aluminum mixture, magnesium/indium mixture, aluminum/aluminum oxide (Al₂O₃) mixture, indium, lithium/aluminum mixture, and a rare earth metal. Among these, from the viewpoint of electron injection properties and durability against oxidation or the like, mixtures of an electron injecting metal and a second metal as a stable metal having a larger work function value than the electron injecting metal, for example, a magnesium/silver mixture, a magnesium/aluminum mixture, a magnesium/indium mixture, an aluminum/aluminum oxide(Al₂O₃) mixture, and a lithium/aluminum mixture, and aluminum are suitable. The cathode can be produced by forming a thin film of such electrode material by a method such as vapor deposition and sputtering. The sheet resistance of the cathode is preferably several hundreds **Ω**/sq or less, and the film thickness is usually selected in the range of 10 nm to 5 **µ**m, preferably 50 to 200 nm. In order to transmit the emitted light, it is favorable that any one of the anode and the cathode of the organic electroluminescent device is transparent or translucent for improvement in the emission luminance.

Further, a transparent or translucent cathode can be produced by producing the metal having a film thickness of 1 to 20 nm on the cathode, and then producing the conductive transparent material in the description of the anode thereon. By applying this, a device with both of the anode and the cathode having transparency can be produced.

### -Light emitting layer-

The light emitting layer is a layer that emits light after excitons are generated by recombination of holes and electrons injected from each of the anode and the cathode, including an organic light emitting material and a host material.

When the light emitting layer is a fluorescence emitting layer, at least one fluorescence emitting material may be used alone as the fluorescence emitting material. However, it is preferable that the light emitting layer include a host material, with use of the fluorescence emitting material as a fluorescence emitting dopant.

As the fluorescence emitting material in the light emitting layer, an oligopyridine compound represented by the general formula (1) can be used, or compounds known from many patent literatures and the like also may be selected for use. Examples thereof include benzoxazole derivatives, benzothiazole derivatives, benzoimidazole derivatives, styrylbenzene derivatives, polyphenyl derivatives, diphenylbutadiene derivatives, tetraphenylbutadiene derivatives, naphthalimide derivatives, coumarin derivatives, condensed aromatic compounds, perinone derivatives, oxadiazole derivatives, oxazine derivatives, aldazine derivatives, cyclopentadiene derivatives, bisstyrylanthracene derivatives, quinacridone derivatives, pyrrolopyridine derivatives, thiadiazolopyridine derivatives, styrylamine derivatives, diketopyrrolopyrrole derivatives, aromatic dimethyridine compounds, metal complexes of 8-quinolinol derivatives, metal complexes of pyrromethene derivatives, rare earth complexes, various metal complexes typified by transition metal complexes, polymer compounds such as polythiophene, polyphenylene and polyphenylene vinylene, and organic silane derivatives. Preferred examples include condensed aromatic compounds, styryl compounds, diketopyrrolopyrrole compounds, oxazine compounds, pyrromethene metal complexes, transition metal complexes and lanthanoid complexes, and more preferred examples include naphthacene, pyrene, chrysene, triphenylene, benzo[c]phenanthrene, benzo[a]anthracene, pentacene, perylene, fluoranthene, acenaphthofluoranthene, dibenzo[a,j]anthracene, dibenzo[a,h]anthracene, benzo[a]naphthacene, hexacene, anthanthrene, naphtho[2,1-f]isoquinoline, α-naphthaphenanthridine, phenanthrooxazole, quinolino[6,5-f]quinoline, and benzothiophanthrene. These may have an alkyl group, an aryl group, an aromatic heterocyclic group, or a diarylamino group as a substituent.

As the fluorescent host material in the light emitting layer, an oligopyridine compound represented by the general formula (1) can be used, or compounds known from many patent literatures and the like also may be selected for use. Examples for use include compounds having a condensed aryl ring such as naphthalene, anthracene, phenanthrene, pyrene, chrysene, naphthacene, triphenylene, perylene, fluoranthene, fluorene, and indene and their derivatives, aromatic amine derivatives such as N,N'-dinaphthyl-N,N'-diphenyl-4,4'-diphenyl-1,1'-diamine, tris(8-quinolinate)aluminum(III) and other metal chelated oxynoid compounds, bisstyryl derivatives such as distyrylbenzene derivatives, tetraphenyl butadiene derivatives, indene derivatives, coumarin derivatives, oxadiazole derivatives, pyrrolopyridine derivatives, perinone derivatives, cyclopentadiene derivatives, pyrrolopyrrole derivatives, thiadiazolopyridine derivatives, dibenzofuran derivatives, carbazole derivatives, indolocarbazole derivatives, triazine derivatives, and polymers such as a polyphenylene vinylene derivatives, polyparaphenylene derivatives, polyfluorene derivatives, polyvinylcarbazole derivatives, and polythiophene derivatives, though not particularly limited thereto.

When the fluorescence emitting material used as the fluorescence emitting dopant contains a host material, the amount of the fluorescence emitting dopant contained in the light emitting layer is in the range of 0.01 to 20 wt%, preferably in the range of 0.1 to 10 wt%.

Usually, an organic electroluminescent device injects electric charges into a light emitting substance from both the anode and cathode electrodes to generate a light emitting substance in excited state, so that light emission is caused. In the case of a charge injection type organic electroluminescent device, it is said that 25% of the generated excitons are excited to a singlet excited state, and the remaining 75% are excited to the triplet excited state. As shown in Advanced Materials 2009, 21, 4802-4806, it is known that a specific fluorescence emitting material allows energy transition to a triplet excited state by intersystem crossing or the like, and then undergoes inverse intersystem crossing to a singlet excited state due to triplet-triplet annihilation or heat energy absorption so as to emit fluorescence, exhibiting thermally activated delayed fluorescence. The organic electroluminescent device of the present invention also can exhibit delayed fluorescence. In this case, both fluorescence emission and delayed fluorescence emission can be included. However, a part of the light may be emitted from the host material, or emission may be partially from the host material.

When the light emitting layer is a delayed fluorescence emitting layer, at least one delayed light emitting material may be used alone, though it is preferable to use the delayed light emitting material as delayed fluorescence emitting dopant and include a host material.

As the delayed fluorescence emitting material in the light emitting layer, the oligopyridine compound represented by the general formula (1) may be used in the case of having a small energy difference between the singlet level and the triplet level, or alternatively, one selected from known delayed fluorescence emitting materials may be used. Examples thereof include tin complexes, indolocarbazole derivatives, copper complexes, and carbazole derivatives. Specific examples thereof include compounds described in the following non patent literature and patent literature, though the present invention is not limited to these compounds.
1) Adv. Mater. 2009, 21, 4802-4806, 2) Appl. Phys. Lett. 98, 083302 (2011), 3) Japanese Patent Laid-Open No. 2011-213643, 4) J. Am. Chem. Soc. 2012, 134, 14706-14709.

Specific examples of the delayed light emitting material are shown below, though not limited to the following compounds.

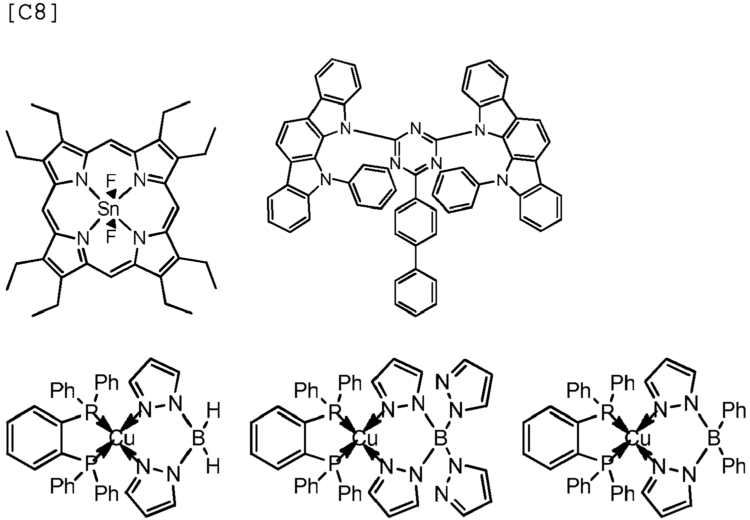

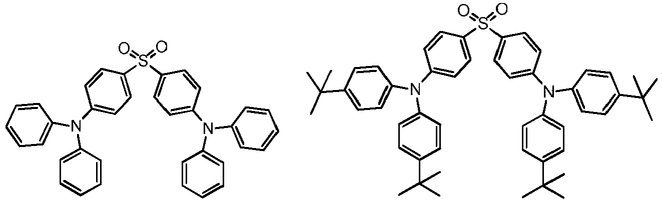

In the case of using the delayed fluorescence emitting material as the delayed fluorescence emitting dopant with a host material included, the amount of the delayed fluorescence emitting dopant contained in the light emitting layer may be in the range of 0.01 to 50 wt%, preferably in the range of 0.1 to 20 wt%, and more preferably in the range of% 0.01 to 10 wt%.

As the delayed fluorescence host material in the light emitting layer, an oligopyridine compound represented by the general formula (1) may be used, or alternatively a compound other than oligopyridine also may be selected for use. Examples for use include compounds having a condensed aryl ring such as naphthalene, anthracene, phenanthrene, pyrene, chrysene, naphthacene, triphenylene, perylene, fluoranthene, fluorene, and indene and their derivatives, aromatic amine derivatives such as N,N'-dinaphthyl-N,N'-diphenyl-4,4'-diphenyl-1,1'-diamine, tris(8-quinolinate)aluminum(III) and other metal chelated oxynoid compounds, bisstyryl derivatives such as distyrylbenzene derivatives, tetraphenyl butadiene derivatives, indene derivatives, coumarin derivatives, oxadiazole derivatives, pyrrolopyridine derivatives, perinone derivative, cyclopentadiene derivative, pyrrolopyrrole derivatives, thiadiazolopyridine derivatives, dibenzofuran derivatives, carbazole derivatives, indolocarbazole derivatives, triazine derivatives, and polymers such as a polyphenylene vinylene derivatives, polyparaphenylene derivatives, polyfluorene derivatives, polyvinylcarbazole derivatives, polythiophene derivatives, and arylsilane derivatives, though not particularly limited thereto.

When the light emitting layer is a phosphorescence emitting layer, the light emitting layer contains a phosphorescence emitting dopant and a host material. The phosphorescence emitting dopant material preferably contains an organic metal complex containing at least one metal selected from ruthenium, rhodium, palladium, silver, rhenium, osmium, iridium, platinum and gold.

Preferred examples of the phosphorescence emitting dopant include complexes having a noble metal element such as Ir as central metal such as Ir(ppy)₃, complexes such as Ir(bt)₂·acac₃, and complexes such as PtOEt₃. Specific examples of these complexes are shown below, though not limited to the following compounds.

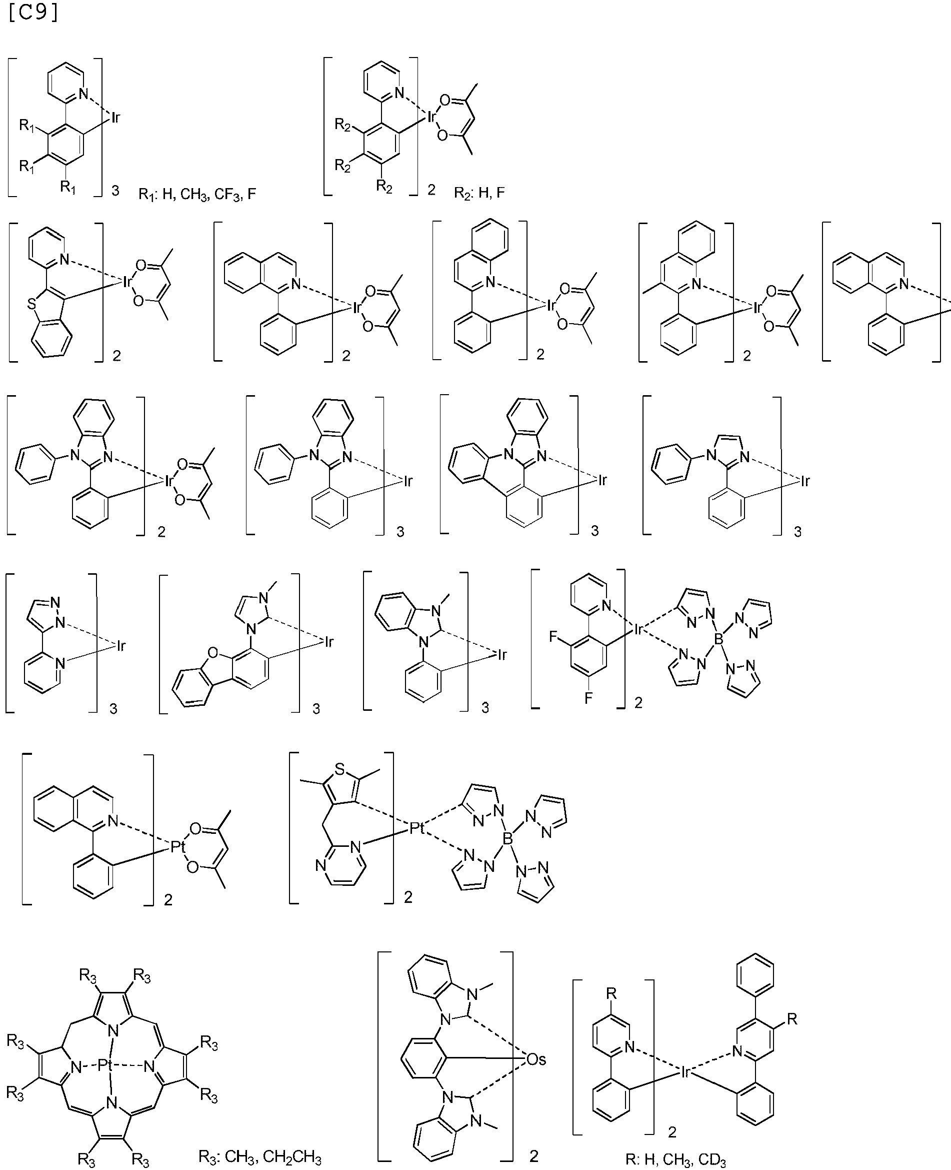

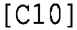

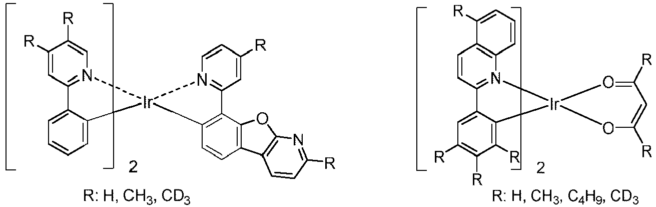

The amount of the phosphorescence emitting dopant contained in the light emitting layer is preferably in the range of 2 to 40 wt%, preferably in the range of 5 to 30 wt%.

When the light emitting layer is a phosphorescence emitting layer, it is preferable to use the oligopyridine compound of the present invention as the host material in the light emitting layer. However, when the oligopyridine compound is used for any organic layer other than the light emitting layer, the material used for the light emitting layer may be a host material other than the oligopyridine compound. Alternatively, the oligopyridine compound may be used in combination with another host material. Further, a plurality of known host materials may be used in combination.

The known host compound that may be used is preferably a compound that has hole transporting ability and electron transporting ability, prevents the wavelength of light emission from being lengthened, and has a high glass transition temperature.

The other host material may be selected from those known from a large number of patent literatures and the like. Specific examples of the host material are not particularly limited, and examples thereof include indole derivatives, carbazole derivatives, indolocarbazole derivatives, triazole derivatives, oxazole derivatives, oxadiazole derivatives, imidazole derivatives, polyarylalkane derivatives, pyrazoline derivatives, pyrazolone derivatives, phenylenediamine derivatives, arylamine derivatives, amino-substituted chalcone derivatives, styrylanthracene derivatives, fluorenone derivatives, hydrazone derivatives, stilbene derivatives, silazane derivatives, aromatic tertiary amine compounds, styrylamine compounds, aromatic dimethylidene compounds, porphyrin-based compounds, anthraquinodimethane derivatives, anthrone derivatives, diphenylquinone derivatives, thiopyrandioxide derivatives, heterocyclic tetracarboxylic acid anhydrides such as naphthalene perylene, phthalocyanine derivatives, metal complexes of 8-quinolinol derivatives and metal phthalocyanine, various metal complexes typified by metal complexes of benzoxazole and benzothiazole derivatives, polymer compounds such as polysilane-based compounds, poly(N-vinylcarbazole) derivatives, aniline-based copolymers, thiophene oligomers, polythiophene derivatives, polyphenylene derivatives, polyphenylene vinylene derivatives, and polyfluorene derivatives.

The light emitting layer may be any of a fluorescence emitting layer, a delayed fluorescence emitting layer, and a phosphorescence emitting layer, and a phosphorescence emitting layer is preferred.

### -Injection layer-

The injection layer is a layer provided between an electrode and an organic layer in order to reduce the drive voltage and improve the emission luminance, including a hole injecting layer and an electron injecting layer, which may be present between the anode and the light emitting layer or the hole transporting layer, and between the cathode and the light emitting layer or the electron transporting layer. The injection layer may be provided on an as needed basis.

### -Hole blocking layer-

The hole blocking layer has the function of the electron transporting layer in a broad sense, being made of hole blocking material having a function of transporting electrons with significantly small hole transporting ability. The hole blocking layer blocks holes while transporting electrons, so that the recombination probability of electrons and holes can be improved.

It is preferable to use the oligopyridine compound of the present invention for the hole blocking layer. However, when the oligopyridine compound is used for any other organic layer, a known hole blocking layer material may be used. Further, as the hole blocking layer material, an electron transporting layer material described later may be used on an as needed basis.

### -Electron blocking layer-

The electron blocking layer is made of a material having a function of transporting holes with a significantly small electron transporting ability. The electron blocking layer blocks electrons while transporting holes, so that the recombination probability of electrons and holes can be improved.

As the material of the electron blocking layer, the material of the hole transporting layer described later may be used on an as needed basis. The film thickness of the electron blocking layer is preferably 3 to 100 nm, more preferably 5 to 30 nm.

### -Exciton blocking layer-

The exciton blocking layer is a layer for preventing excitons generated by the recombination of holes and electrons in the light emitting layer from diffusing into the charge transporting layer. Insertion of the layer enables the excitons to be efficiently confined in the light emitting layer, so that the luminous efficiency of the device can be improved. The exciton blocking layer may be inserted into any one of the anode side and the cathode side adjacent to the light emitting layer, or may be inserted into both at the same time.

Although an oligopyridine compound represented by the general formula (1) may be used as the material of the exciton blocking layer, examples of other materials for use include 1,3-dicarbazolylbenzene(mCP) and bis(2-methyl-8-quinolinolato)-4-phenylphenylatoaluminum (III) (BAlq) .

### -Hole transporting layer-

The hole transporting layer is made of a hole transporting material having a function of transporting holes, being provided as a single layer or a plurality of layers.

The hole transporting material has any of hole injecting or transporting properties and electron barrier properties, and may be any of an organic substance or an inorganic substance. Although it is preferable to use an oligopyridine compound represented by the general formula (1) as a known hole transporting material that can be used, any conventionally known compound may be selected for use. The known hole transporting materials that can be used include triazole derivatives, oxadiazole derivatives, imidazole derivatives, polyarylalkane derivatives, pyrazoline derivatives and pyrazolone derivatives, phenylenediamine derivatives, arylamine derivatives, amino-substituted chalcone derivatives, oxazole derivatives, styrylanthracene derivatives, fluorenone derivatives, hydrazone derivatives, stilbene derivatives, silazane derivatives, aniline-based copolymers, and conductive polymer oligomers, especially thiophene oligomers. Use of porphyrin compounds, aromatic tertiary amine compounds or styrylamine compounds is preferred, and use of aromatic tertiary amine compounds is more preferred.

### -Electron transporting layer-

The electron transporting layer is made of a material having a function of transporting electrons, which may be provided with a single layer or a plurality of layers.

The electron transporting material (which may also serve as a hole blocking material) has only to have a function of transmitting electrons injected from the cathode to the light emitting layer. Although it is preferable to use the oligopyridine compound of the present invention for the electron transporting layer, any of conventionally known compounds may be selected for use. Examples thereof include nitro-substituted fluorene derivatives, diphenylquinone derivatives, thiopyrandioxide derivatives, carbodiimides, fluorenylidenemethane derivatives, anthraquinodimethane and anthrone derivatives, and oxadiazole derivatives. Further, among the oxadiazole derivatives, thiadiazole derivatives with an oxygen atom of the oxadiazole ring replaced with a sulfur atom, and quinoxaline derivatives having a quinoxaline ring known as electron-withdrawing group also may be used as electron transporting material. Further, polymer materials with these materials introduced into a polymer chain, or with these materials as polymer main chain, also may be used.

### Examples

Hereinafter, the present invention will be described in more detail with reference to Examples, though the present invention is, of course, not limited to these Examples, and can be performed in various forms within the scope of the invention.

An oligopyridine compound for use as material for organic electroluminescent devices was synthesized by the route shown below. The compound number corresponds to the number assigned to the chemical formula described above.

### Example 1 (Synthesis Example)

A compound 1-1 was synthesized according to the following reaction formula.

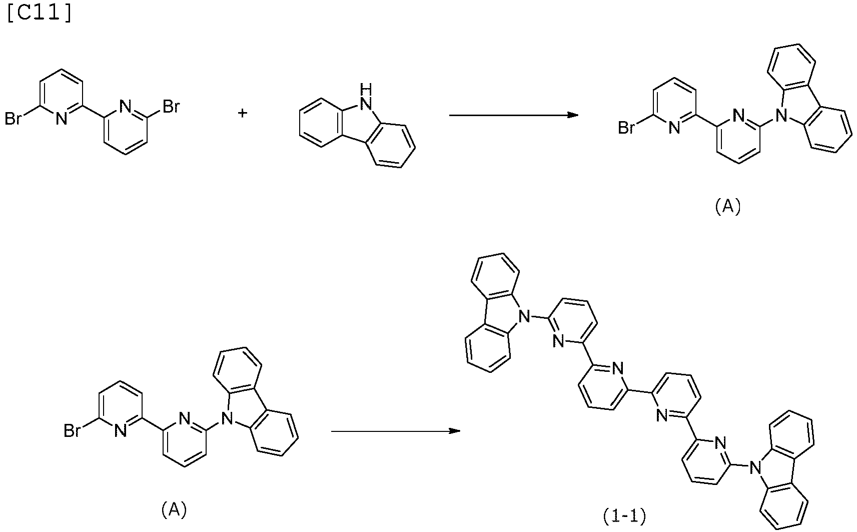

Under a nitrogen atmosphere, 5.0 g (0.0159 mol) of 6,6'-dibromo-2,2'-bipyridine, 2.6 g (0.0159 mol) of carbazole, 1.5 g (7.95 mmol) of copper iodide, 10.1 g (0.0477 mol) of tripotassium phosphate, 1.9 mL (0.0159 mol) of trans-1,2-cyclohexanediamine and 100 mL of 1,4-dioxane were added, and the mixture was stirred overnight at 115°C. After cooling the reaction solution to room temperature, the precipitated crystals were collected by filtration and the solvent was distilled away under reduced pressure. The obtained residue was purified by silica gel column chromatography to obtain 3.0 g (7.49 mmol, yield: 47%) of an intermediate (A) (APCI-TOFMS, m/z 401 [M+H]⁺).

After 1.8 g (7.49 mmol) of nickel(II) chloride hexahydrate, 9.0 g (34.4 mmol) of triphenylphosphine and 100 mL of DMF were added and dissolved, the mixture was sufficiently degassed under reduced pressure. Then, the inside of the container was replaced with nitrogen, and the mixture was stirred at 60°C for 1 hour. Here, 0.7 g (10.5 mmol) of zinc powder was added, and the mixture was stirred again at 60°C for 1 hour. To the resulting brown solution, 3.0 g (7.49 mmol) of the intermediate (A) was added, and the mixture was stirred at 90°C overnight. After cooling the reaction solution to room temperature, 500 mL of 25% aqueous ammonia was added thereto and the precipitated gray solid was collected by filtration. The resulting gray solid was purified by silica gel column chromatography to obtain 2.8 g (4.49 mmol, yield: 60%) of the compound 1-1 (APCI-TOFMS, m/z 641[M+H]⁺).

### Example 2 (Synthesis Example)

A compound 1-35 was synthesized according to the following reaction formula.

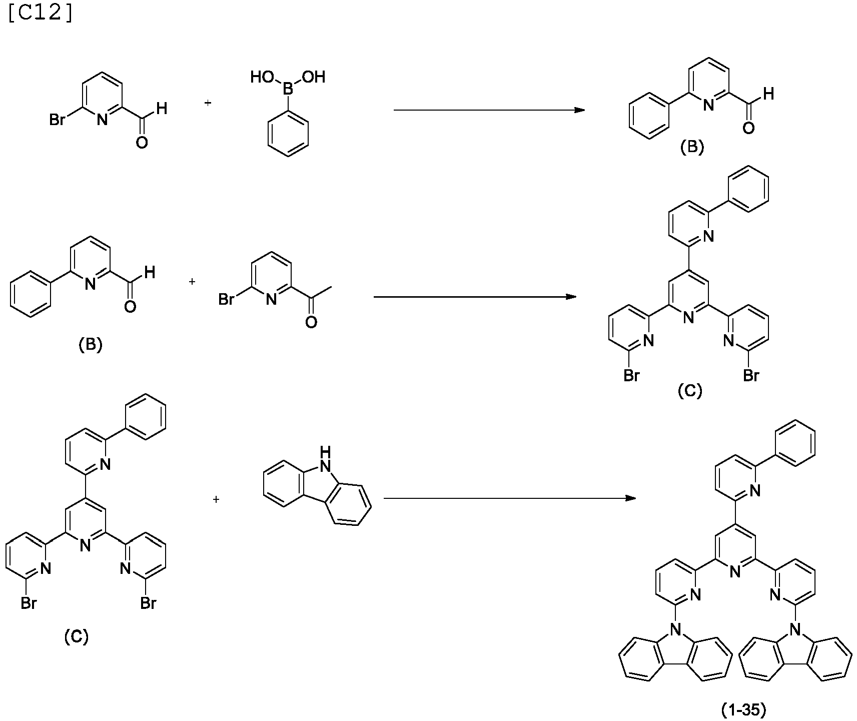

Under a nitrogen atmosphere, 2.6 g (14.1 mmol) of 6-bromo-2-pyridinecarboxyaldehyde, 1.7 g (14.1 mmol) of phenylboronic acid, 0.407 g (0.352 mmol) of tetrakis (triphenylphosphine)palladium (0), 13.7 g (42.3 mmol) sodium carbonate, and 100 ml of 1,4-dioxane were added, and the mixture was stirred for 1 hour while heating at 115°C. After cooling the reaction solution to room temperature, the precipitated crystals were collected by filtration and the solvent was distilled away under reduced pressure. The obtained residue was purified by silica gel column chromatography to obtain 2.0 g (10.9 mmol, yield: 77%) of an intermediate (B) (APCI-TOFMS, m/z 184 [M+H]⁺).

Under a nitrogen atmosphere, 7.0 g (38.2 mmol) of the intermediate (B), 15.3 g (76.4 mmol) of 6-bromo-2-acetylpyridine, 2.14 g (38.2 mmol) of potassium hydroxide, 250 mL of 25% aqueous ammonia, and 764 mL of ethanol were stirred overnight while heating at 85°C. After cooling the reaction solution to room temperature, the precipitated solid was collected by filtration, and the resulting solid was purified by silica gel column chromatography to obtain 3.6 g (6.61 mmol, yield: 17%) of an intermediate (C) (APCI-TOFMS, m/z 545[M+H]⁺).

Under a nitrogen atmosphere, 3.6 g (6.61 mmol) of intermediate (C), 2.7 g (15.9 mmol) of carbazole, 0.3 g (1.59 mmol) of copper iodide, 5.61 g (26.4 mmol) of tripotassium phosphate, 2.0 mL (15.9 mmol) of trans-1,2-cyclohexanediamine, and 100 mL of 1,4-dioxane were added, and the mixture was stirred at 115°C overnight. After cooling the reaction solution to room temperature, the precipitated crystals were collected by filtration and the solvent was distilled away under reduced pressure. The resulting residue was purified by silica gel column chromatography to obtain 3.2 g (4.46 mmol, yield: 67%) of the compound 1-35 (APCI-TOFMS, m/z 717[M+H]⁺).

In addition to compounds 1-1 and 1-35, compounds 1-32, 1-33, 1-34, 1-36, 1-70, 1-83 and 1-84 were synthesized according to the above synthesis example. In addition, compounds H-1, H-2 and H-3 were synthesized for comparison.

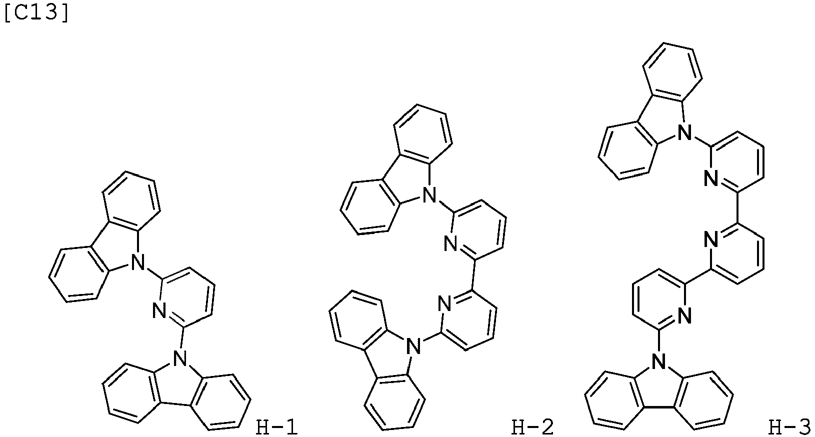

### Example 3

On a glass substrate having an anode made of ITO with a film thickness of 110 nm thereon, each thin film was laminated with a vacuum degree of 4.0×10⁻⁵ Pa by a vacuum vapor deposition method. First, HAT-CN was formed to a thickness of 25 nm as a hole injecting layer on the ITO, and then NPD was formed to a thickness of 30 nm as a hole transporting layer. Next, HT-1 was formed to a thickness of 10 nm as an electron blocking layer. Next, the compound 1-1 as host material and Ir(ppy)₃ as light emitting dopant were co-deposited from different vapor deposition sources to form a light emitting layer to a thickness of 40 nm. At this time, the concentration of Ir(ppy)₃ was 10 wt%. Furthermore, ET-2 was formed to a thickness of 5 nm as a hole blocking layer. Next, ET-1 was formed to a thickness of 15 nm as an electron transporting layer. Further, LiF was formed to a thickness of 1 nm on the electron transport layer as an electron injecting layer. Finally, Al was formed to a thickness of 70 nm as a cathode on the electron injecting layer to make an organic electroluminescence device.

When an external power source was connected to the obtained organic electroluminescence device to apply a DC voltage, an emission spectrum with a maximum wavelength of 517 nm was observed, so that it has been found that emission from Ir(PPy)₃ was obtained.

### Examples 4 to 10

An organic electroluminescence device was produced in the same manner as in Example 3, except that compound 1-32, 1-33, 1-34, 1-35, 1-36, 1-70, or 1-83 was used instead of the compound 1-1 as the host material for the light emitting layer in Example 3. When an external power source was connected to the obtained organic electroluminescence device to apply a DC voltage, an emission spectrum with a maximum wavelength of 517 nm was observed, so that it has been found that emission from Ir(PPy)₃ was obtained.

### Comparative Examples 1 to 3

An organic electroluminescence device was produced in the same manner as in Example 3, except that H-1, H-2 or H-3 was used as the host material for the light emitting layer in Example 3. When an external power source was connected to the obtained organic electroluminescence device to apply a DC voltage, an emission spectrum with a maximum wavelength of 517 nm was observed.

The evaluation results of the produced organic electroluminescence devices are shown in Table 1. In the table, the luminance, drive voltage, and luminous efficiency are the values when the drive current is 20 mA/cm², representing the initial characteristics. LT70 is the time required for the initial luminance to decay to 70%, representing the life characteristics.

**[Table 1]**

| | Host material compound | Luminance (cd/m²) | Voltage (V) | Power efficiency (l/W) | LT70 (h) |
|---|---|---|---|---|---|
| Ex. 3 | 1-1 | 7000 | 3.6 | 30.5 | 600 |
| Ex. 4 | 1-32 | 7000 | 3.4 | 32.3 | 600 |
| Ex. 5 | 1-33 | 7000 | 3.3 | 33.3 | 600 |
| Ex. 6 | 1-34 | 8000 | 3.3 | 38.1 | 700 |
| Ex. 7 | 1-35 | 7000 | 3.2 | 34.4 | 650 |
| Ex. 8 | 1-36 | 7000 | 3.1 | 35.5 | 600 |
| Ex. 9 | 1-70 | 8000 | 3.0 | 41.9 | 600 |
| Ex. 10 | 1-83 | 7000 | 3.6 | 30.5 | 750 |
| Comp. Ex. 1 | H-1 | 8000 | 4.8 | 26.2 | 300 |
| Comp. Ex. 2 | H-2 | 7000 | 4.3 | 25.6 | 450 |
| Comp. Ex. 3 | H-3 | 7000 | 4.1 | 26.8 | 350 |

### Example 11

On a glass substrate having an anode made of ITO with a film thickness of 110 nm thereon, each thin film was laminated with a vacuum degree of 4.0×10⁻⁵ Pa by a vacuum vapor deposition method. First, HAT-CN was formed to a thickness of 25 nm as a hole injecting layer on the ITO, and then NPD was formed to a thickness of 45 nm as a hole transporting layer. Next, HT-1 was formed to a thickness of 10 nm as an electron blocking layer. Then, the compound 1-1 as host material and Ir(piq)₂acac as light emitting dopant were co-deposited from different vapor deposition sources to form a light emitting layer to a thickness of 40 nm. At this time, the concentration of Ir(piq)₂acac was 6.0 wt%. Furthermore, ET-2 was formed to a thickness of 10 nm as a hole blocking layer. Next, ET-1 was formed to a thickness of 27.5 nm as an electron transporting layer. Then, LiF was formed to a thickness of 1 nm on the electron transporting layer as an electron injecting layer. Finally, Al was formed to a thickness of 70 nm as a cathode on the electron injecting layer to make an organic electroluminescence device. When an external power source was connected to the obtained organic electroluminescence device to apply a DC voltage, an emission spectrum with a maximum wavelength of 620 nm was observed, so that it has been found that emission from Ir(piq)₂acac was obtained.

### Examples 12 to 18

An organic electroluminescence device was produced in the same manner as in Example 11, except that compound 1-32, 1-33, 1-34, 1-35, 1-36, 1-70, or 1-83 was used instead of the compound 1-1 as the host material for the light emitting layer in Example 11. When an external power source was connected to the obtained organic electroluminescence device to apply a DC voltage, an emission spectrum with a maximum wavelength of 620 nm was observed, so that it has been found that emission from Ir(piq)₂acac was obtained.

### Comparative Examples 4 to 6

An organic electroluminescence device was produced in the same manner as in Example 11, except that H-1, H-2 or H-3 was used as the host material for the light emitting layer in Example 11. When an external power source was connected to the obtained organic electroluminescence device to apply a DC voltage, an emission spectrum with a maximum wavelength of 620 nm was observed.

The evaluation results of the produced organic electroluminescence devices are shown in Table 2. LT95 is the time required for the initial luminance to decay to 95%, representing the life characteristics.

**[Table 2]**

| | Host material compound | Luminance (cd/m²) | Voltage (V) | Power efficiency (l/W) | LT95 (h) |
|---|---|---|---|---|---|
| Ex. 11 | 1-1 | 4500 | 3.5 | 20.2 | 250 |
| Ex. 12 | 1-32 | 4500 | 3.3 | 21.4 | 250 |
| Ex. 13 | 1-33 | 4500 | 3.2 | 22.1 | 250 |
| Ex. 14 | 1-34 | 5000 | 3.2 | 24.5 | 300 |
| Ex. 15 | 1-35 | 4500 | 3.2 | 22.1 | 250 |
| Ex. 16 | 1-36 | 4500 | 3.1 | 22.8 | 250 |
| Ex. 17 | 1-70 | 5000 | 3.1 | 25.3 | 250 |
| Ex. 18 | 1-83 | 4500 | 3.5 | 20.2 | 350 |
| Comp.Ex. 4 | H-1 | 5000 | 4.6 | 17.1 | 100 |
| Comp. Ex. 5 | H-2 | 4500 | 4.4 | 16.1 | 150 |
| Comp. Ex. 6 | H-3 | 4500 | 4.0 | 17.7 | 120 |

From Tables 1 and 2, it can be seen that in Examples 3 to 18, good characteristics are exhibited with improved power efficiency and life characteristics.

### Example 19

On a glass substrate having an anode made of ITO with a film thickness of 110 nm thereon, each thin film was laminated with a vacuum degree of 4.0×10⁻⁵ Pa by a vacuum vapor deposition method. First, HAT-CN was formed to a thickness of 25 nm as a hole injection layer on the ITO, and then NPD was formed to a thickness of 30 nm as a hole transporting layer. Next, HT-1 was formed to a thickness of 10 nm as an electron blocking layer. Next, the compound H-2 as host material and Ir(ppy)₃ as light emitting dopant were co-deposited from different vapor deposition sources to form a light emitting layer to a thickness of 40 nm. At this time, the concentration of Ir(ppy)₃ was 10 wt%. Furthermore, the compound 1-1 was formed to a thickness of 5 nm as a hole blocking layer. Next, ET-1 was formed to a thickness of 15 nm as an electron transporting layer. Further, LiF was formed to a thickness of 1 nm on the electron transport layer as an electron injecting layer. Finally, Al was formed to a thickness of 70 nm as a cathode on the electron injection layer to make an organic electroluminescence device.

### Examples 20 to 26

An organic electroluminescence device was produced in the same manner as in Example 19, except that compound 1-32, 1-33, 1-34, 1-35, 1-36, 1-70, or 1-83 was used instead of the compound 1-1 as the hole blocking layer in Example 19.

### Comparative Examples 7 to 9

An organic electroluminescence device was produced in the same manner as in Example 19, except that H-1, H-2 or H-3 was used as the hole blocking layer for the light emitting layer in Example 19.

The evaluation results of the produced organic electroluminescence devices are shown in Table 3.

**[Table 3]**

| | Hole blocking layer compound | Luminance (cd/m²) | Voltage (V) | Power efficiency (l/W) | LT70 (h) |
|---|---|---|---|---|---|
| Ex. 19 | 1-1 | 8000 | 3.8 | 33.1 | 700 |
| Ex. 20 | 1-32 | 8000 | 3.5 | 35.9 | 700 |
| Ex. 21 | 1-33 | 8000 | 3.4 | 37.0 | 700 |
| Ex. 22 | 1-34 | 8500 | 3.4 | 39.3 | 800 |
| Ex. 23 | 1-35 | 8000 | 3.3 | 38.1 | 700 |
| Ex. 24 | 1-36 | 8000 | 3.2 | 39.3 | 700 |
| Ex. 25 | 1-70 | 8500 | 3.1 | 43.1 | 700 |
| Ex. 26 | 1-83 | 8000 | 3.7 | 34.0 | 850 |
| Comp. Ex. 7 | H-1 | 8000 | 4.9 | 25.6 | 350 |
| Comp. Ex. 8 | H-2 | 7500 | 4.6 | 25.6 | 550 |
| Comp. Ex. 9 | H-3 | 7500 | 4.6 | 25.6 | 400 |

The compounds used in Examples are shown below.

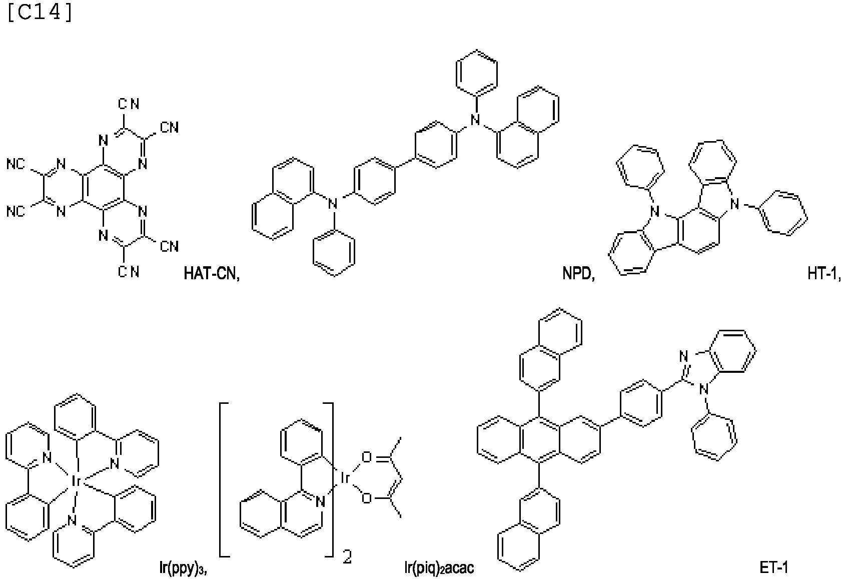

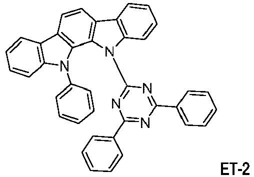

### Industrial Applicability

Since the material for an organic electroluminescent device of the present invention exhibits good amorphous properties and high thermal stability and is extremely stable in an excited state, an organic electroluminescent device using the same has a long drive life and durability at a practical level.

## Claims

1. A material for organic electroluminescent devices comprising an oligopyridine compound represented by the following general formula (1): 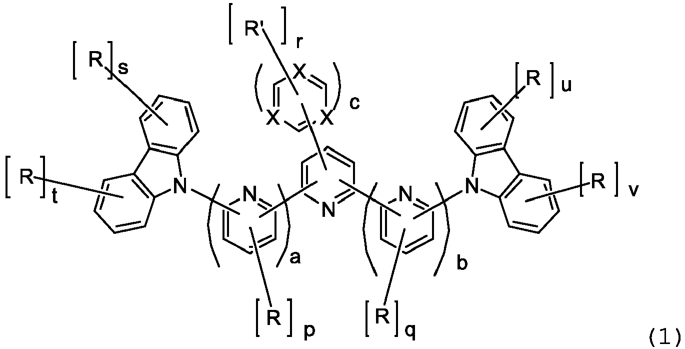 wherein, R and R' each are independently hydrogen, deuterium, an aliphatic hydrocarbon group having 1 to 10 carbon atoms, a substituted or unsubstituted aromatic hydrocarbon group having 6 to 10 carbon atoms, or a substituted or unsubstituted aromatic heterocyclic group having 3 to 12 carbon atoms, and when R and R' each are an aromatic hydrocarbon group or an aromatic heterocyclic group, it may form a condensed ring with a ring to be substituted by R or R', provided that R' contains no 9-carbazole ring group; X independently represents N, C-R' or C-, and at least one is N; a, b and c each represent the number of repetitions and each independently represent an integer of 0 to 3, and a+b+c≥3; p, q, r, s, t, u and v each represent the number of substitutions and each independently represent an integer of 1 to 3.

2. The material for organic electroluminescent devices according to claim 1, wherein the compound represented by the general formula (1) is a compound represented by the following formula (2), (3) or (4): 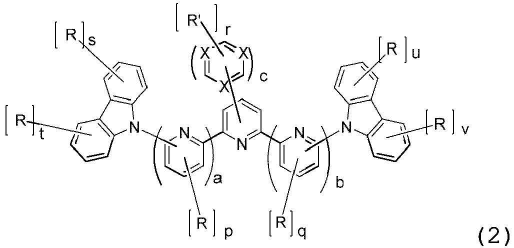 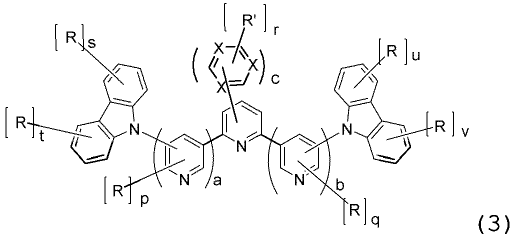 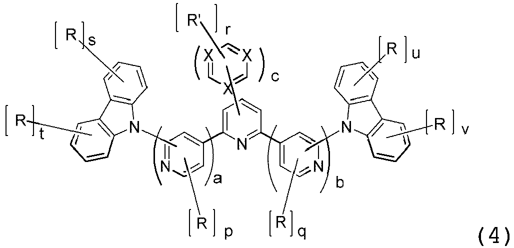 wherein X, R, R', a, b, c, p, q, r, s, t, u and v are the same as in the general formula (1).

3. The material for organic electroluminescent devices according to claim 2, wherein a+b=3 in the formulas (2) to (4) .

4. An organic electroluminescent device comprising an anode, organic layers and a cathode laminated on a substrate, wherein at least one layer of the organic layers is an organic layer containing the material for organic electroluminescent devices according to claims 1 to 3.

5. The organic electroluminescent device according to claim 4, wherein the organic layer containing the material for organic electroluminescent devices may be at least one layer selected from the group consisting of a light emitting layer, an electron transporting layer, and a hole blocking layer.

6. The organic electroluminescent device according to claim 4, wherein the organic layer containing the material for organic electroluminescent devices is a light emitting layer containing a host material and a light emitting dopant material.

7. The organic electroluminescent device according to claim 6, wherein the light-emitting dopant material is an organic metal complex containing at least one metal selected from the group consisting of ruthenium, rhodium, palladium, silver, rhenium, osmium, iridium, platinum and gold.

8. The organic electroluminescent device according to claim 6, wherein the light-emitting dopant material is a thermally activated delayed fluorescence emitting dopant.

9. The organic electroluminescent device according to any one of claims 4 to 8, wherein a hole blocking layer is provided adjacent to the light emitting layer, and the hole blocking layer contains the material for organic electroluminescent devices.
